(19) Europäisches Patentamt
European Patent Office
Office européen des brevets

(11) Publication number : **0 557 167 A1**

# (12) EUROPEAN PATENT APPLICATION

(21) Application number : **93400360.9**

(22) Date of filing : **12.02.93**

(51) Int. Cl.$^5$ : **C07C 49/167,** C07C 59/115, C07C 69/708, C07C 59/135, C07C 31/44, C07C 59/21, C07C 41/42, C07C 45/54, C07C 51/00, C07C 29/09, C07C 67/08, // C07C59/215, C07C49/643, C07B39/00, C07B41/00, C07C69/96, C07C68/06, C07D317/42, C07D317/36

(30) Priority : **12.02.92 US 834614**

(43) Date of publication of application : **25.08.93 Bulletin 93/34**

(84) Designated Contracting States : **DE FR GB IT**

(71) Applicant : **MINNESOTA MINING AND MANUFACTURING COMPANY**
**3M Center, P.O. Box 33427**
**St. Paul, Minnesota 55133-3427 (US)**

(72) Inventor : **Richman, Jack E., c/o Minnesota Mining and Manufacturing Co., 2501 Hudson Road, PO Box 33427**
**St. Paul, Minnesota 55133-3427 (US)**

(74) Representative : **Warcoin, Jacques et al**
**Cabinet Régimbeau 26, avenue Kléber**
**F-75116 Paris (FR)**

## (54) Preparation of fluorinated functional compounds.

(57) Fluorinated functional compounds, namely perfluorinated and partially-fluorinated functional compounds, such as perfluorinated and partially-fluorinated carboxylic acids, esters, ketones, alcohols, amides, carboxylic acid fluorides, and derivatives thereof, are prepared by :

(a) directly fluorinating a fluorinatable cyclic or acyclic carbonate by contacting the carbonate with fluorine gas in a temperature-controlled reactor to fluorinate the carbonate, and

(b) combining the resulting fluorinated carbonate with a reactive nucleophile and allowing the carbonate and the nucleophile to react to form a fluorinated functional derivative of the fluorinated carbonate.

EP 0 557 167 A1

This invention relates to a process for preparing fluorinated functional compounds, for example, perfluorinated and partially-fluorinated carboxylic acids, esters, ketones, amides, alcohols, and carboxylic acid fluorides.

Fluorochemical compounds and their derivatives (sometimes called organofluorine compounds or fluorochemicals) are a class of substances which contain portions that are fluoroaliphatic or fluorocarbon in nature, e.g., nonpolar, hydrophobic, oleophobic, and chemically inert, and which may further contain portions which are functional in nature, e.g., polar and chemically reactive. The class includes some commercial substances which are familiar to the general public, such as those which give oil and water repellency and stain and soil resistance to textiles, e.g., Scotchgard™ carpet protector. Other substances of the class have various industrial uses, such as reducing the surface tension of liquids, reducing evaporation and flammability of volatile organic liquids, improving the leveling of organic polymer coatings, and serving as intermediates in the preparation of rubber curatives. Examples of industrial fluorochemical substances are the Fluorad™ fluorochemical surfactants described in 3M Company trade bulletin 98-0211-2213-4(38.3)BPH, issued March 1988, and hexafluoroacetone, which is used for the preparation of polymer intermediates.

An industrial method of producing many fluorinated compounds, such as perfluorinated and partially-fluorinated organofluorine compounds, is the electrochemical fluorination process, commercialized initially in the 1950s by 3M Company, which comprises passing an electric current through a mixture of the organic starting compound and liquid anhydrous hydrogen fluoride. This fluorination process is commonly referred to as the "Simons electrochemical fluorination process," or more simply either the Simons process or electrochemical fluorination (ECF), and is one method by which fluorinated carboxylic acid fluorides are generally prepared. The fluorinated carboxylic acid fluorides are useful as precursors of fluorinated functional compounds such as fluorinated esters, ketones, amides, and carboxylic acids, as well as reduction products (e.g., dihydroalcohols) derived therefrom. (See, for example, U.S. Pat. Nos. 2,567,011 (Diesslin et al.), 2,666,797 (Husted et al.), and 3,385,904 (Pavlik), which describe the preparation of fluorinated acids, alcohols, esters, and other derivatives by ECF and subsequent reaction steps.) However, a drawback of ECF is that side reactions often occur. Such side reactions involve cleavage of carbon-carbon bonds and polymer formation. (See <u>Encyclopedia of Chemical Technology</u>, Kirk-Othmer, Third Edition, Volume 10, pages 835-36, John Wiley & Sons (1980).) For example, when fluorinated carboxylic acid fluorides are prepared by ECF, low purity and low yields are often obtained due to the formation of rearrangement and cleavage products. International Publication No. WO 90/03353 (Exfluor Research Corporation) accordingly states that carboxylic acids and their derivatives can be fluorinated electrochemically, but that the yields are usually low and have a tendency to decline rapidly as the number of carbons in the molecule is increased. This publication states that, in general, very low yields (less than 25%) will be obtained for any perfluorinated carboxylic acid containing over 12 carbon atoms.

A second method of preparing fluorinated functional compounds is by the telomerization of tetrafluoroethylene to form fluorocarbon intermediates which are subsequently convertible to fluorinated functional compounds such as surfactants. (This method is described by R. E. Banks in <u>Organofluorine Chemicals and their Industrial Applications</u>, pages 217-22, Ellis Horwood Limited, Chichester (1979).) A characteristic of this method is that a distribution of molecular weights is obtained.

In a third method, fluorinated functional compounds can be obtained by direct fluorination of their hydrocarbon counterparts, using fluorine gas. International Publication No. WO 90/03353 (Exfluor Research Corporation) describes a liquid phase fluorination method for perfluorination of a wide variety of hydrogen-containing compounds. The method involves direct fluorination under mild conditions which make possible the preservation of chemical functionalities. Thus, polyesters and acyl fluorides are said to be convertible to perfluorocarbons with essentially complete retention of the ester functionality. International Patent Publication No. WO 90/06296 (Minnesota Mining & Manufacturing Company) discloses an improved liquid phase process of direct fluorination of perfluorinatable organic substances (such as ethers, alcohols, carboxylic acid esters, acid fluorides, sulfonyl fluorides, and sulfonate esters), wherein the fluorination is carried out at a temperature and a flow rate of inert gas (if used) sufficient to volatilize the resulting by-product hydrogen fluoride, removing the hydrogen fluoride from the reactor as it is produced. The process can be carried out to produce high yields of perfluorinated product. International Patent Publication No. WO 89/01929 (Exfluor Research Corporation) describes the direct fluorination of orthocarbonates and polyalkoxypropanes.

U.S. Pat. No. 3,455,954 (Prager) discloses fluorinated cyclic carbonates prepared by direct fluorination of corresponding organic cyclic carbonates. The fluorinated cyclic carbonates are said to be useful as solvents, hydraulic fluids, dielectrics, or, when lower boiling, as refrigerants or aerosol propellants.

Briefly, this invention provides a process for the preparation of fluorinated functional compounds, namely perfluorinated and partially-fluorinated functional compounds, such as perfluorinated and partially-fluorinated carboxylic acids, esters, ketones, alcohols, amides, carboxylic acid fluorides, and derivatives thereof. The process comprises the following steps: (a) directly fluorinating a fluorinatable cyclic or acyclic carbonate by contacting the carbonate with fluorine gas in a temperature-controlled reactor to fluorinate the carbonate; and (b) combining the resulting fluorinated carbonate with a reactive nucleophile and allowing the carbonate and the nucleophile to react to form a fluorinated functional derivative of the fluorinated carbonate. Preferably, the direct fluorination step is carried out by directly contacting a diluted solution or dispersion of a fluorinatable cyclic or acyclic carbonate in a normally liquid, inert medium with fluorine gas, preferably diluted with an inert gas such as nitrogen, in a temperature-controlled reactor to fluorinate the carbonate either in the presence of a hydrogen fluoride scavenger, such as sodium fluoride, or at a temperature and a flow rate of inert gas (if used) sufficient to volatilize the resulting by-product hydrogen fluoride, removing the hydrogen fluoride from the reactor as it is produced (and not recycling it) so that the fluorination is carried out in a substantially hydrogen fluoride-free environment. As used herein, the term "fluorinatable" means having one or more hydrogen atoms replaceable by fluorine.

The process of the invention can be employed as a flexible route to many different fluorinated functional compounds. Perfluorinated and partially-fluorinated carboxylic acids, esters, ketones, alcohols, amides, and carboxylic acid fluorides (as well as perfluorinated and partially-fluorinated multifunctional compounds, for example, diacids, diesters, diketones, keto acids, hydroxy acids, diols, and tetraketones) may be obtained in improved yields and purity and at lower cost than when prepared by conventional methods such as ECF. The use of carbonates in the process of the invention provides a higher efficiency of fluorine use compared to the fluorination of esters such as alkyl acetates (for which part of the fluorine is used to fluorinate the acetyl group which is thereafter cleaved from the molecule) and also provides a higher conversion efficiency relative to the use of other fluorinatable starting materials. Further, the use of carbonates in the process of this invention yields intermediates (fluorocarbonates) which are of higher molecular weight and are less volatile than those which can be obtained by conventional routes. For example, perfluoro(isopropyl carbonate) is a higher molecular weight and less volatile intermediate for the production of hexafluoroacetone than is perfluoro(isopropyl acetate). An additional advantage of the use of carbonate starting materials is their ease and flexibility of preparation, as a number of different preparative methods are available.

A class of fluorinatable cyclic and acyclic carbonates which can be utilized in the process of this invention are monocarbonates which can be represented by the formula R-O-C(O)-O-R', wherein R and R' are independently selected from the group consisting of primary, secondary, and tertiary aliphatic groups having, for example, from 1 to 20 carbon atoms; alkyl-substituted and unsubstituted cycloaliphatic groups having, for example, from 3 to 20 carbon atoms; aryl groups having, for example, from 6 to 14 carbon atoms; alkylene groups having, for example, from 1 to 20 carbon atoms and being such that R and R' are bonded together to form a cyclic structure; and combinations of such groups. R and/or R' can also be partially-fluorinated or partially-chlorinated; R or R' can be fully-fluorinated; R and/or R' can contain one or a plurality of carbon-bonded, catenary heteroatoms (such as catenary oxygen atoms); and R and/or R' can further contain additional carbonate functionality (-O-C(O)-).

A preferred subclass of monocarbonates for use in the process of the invention are those of said formula R-O-C(O)-O-R' wherein R and R' are independently selected from the group consisting of primary, secondary, and tertiary aliphatic groups having from 1 to 12 carbon atoms; alkyl-substituted and unsubstituted cycloaliphatic groups having from 3 to 12 carbon atoms; aryl groups having from 6 to 12 carbon atoms; and alkylene groups having from 1 to 12 carbon atoms and being such that R and R' are bonded together to form a cyclic structure. The R and R' groups of the preferred subclass can contain fluorine, chlorine, catenary heteroatoms, or other carbonate functionality, as described above.

The precursor carbonates, which can be directly fluorinated and then allowed to react with various nucleophiles to form the fluorinated functional compounds of the invention, can be prepared by utilizing known organic reaction techniques. One preparative method of importance involves the reaction of phosgene or its equivalents, e.g., triphosgene, with hydroxy compounds in the presence of a base such as pyridine. Symmetrical cyclic and acyclic carbonates can be prepared using this method. Asymmetrical aliphatic or aromatic-aliphatic cyclic and acyclic carbonates can be prepared by a second method involving condensing a chloroformic acid ester with a hydroxy compound in the presence of a base. A third method of preparation which can be utilized involves the reaction of an oxirane with carbon dioxide in the presence of a catalyst such as an alkyl iodide or an ammonium or phosphonium compound. Cyclic carbonates can be prepared by this method. Examples of these three methods of carbonate synthesis are shown in Schemes 1, 2, and 3 below:

Scheme 1

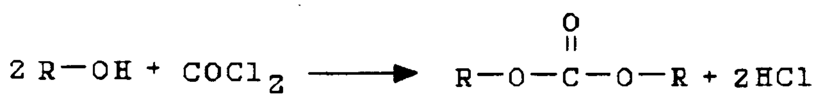

Scheme 2

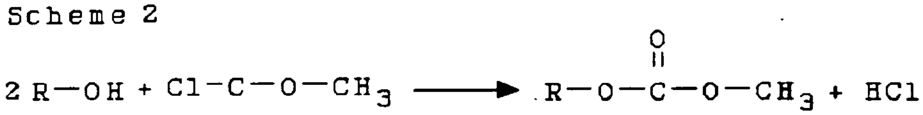

Scheme 3

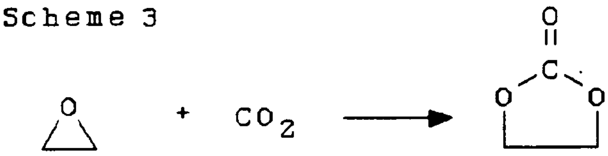

These methods of carbonate preparation, as well as others, are described, for example, in Ullmann's Encyclopedia of Industrial Chemistry, Fifth Edition, Volume A5, pages 198-99, VCH Publishers, New York (1986). Other useful references concerning carbonate synthesis include Kirk-Othmer Encyclopedia of chemical Technology, Third Edition, Volume 4, page 768, John Wiley & Sons, New York (1978) and Rodd's Chemistry of Carbon Compounds, Supplement to Volume 1 of the Second Edition, pages 155-59, Elsevier Scientific Publishing Company, Amsterdam (1973).

Representative examples of precursor carbonates which can be utilized in the process of the invention include the compounds shown below and their partially-fluorinated and partially-chlorinated analogs:

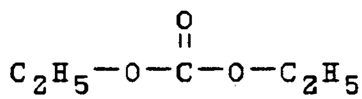

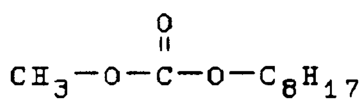

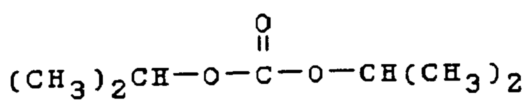

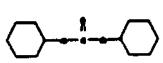

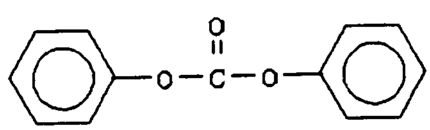

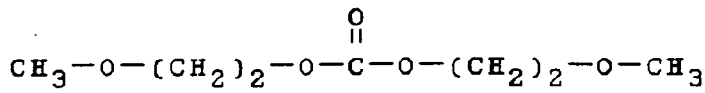

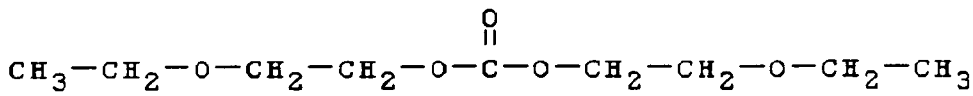

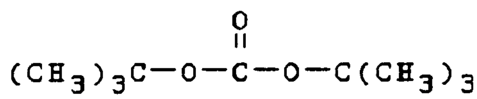

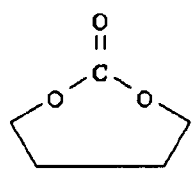

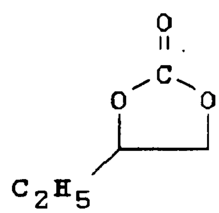

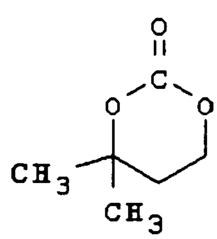

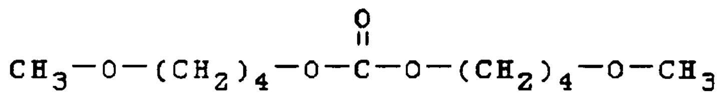

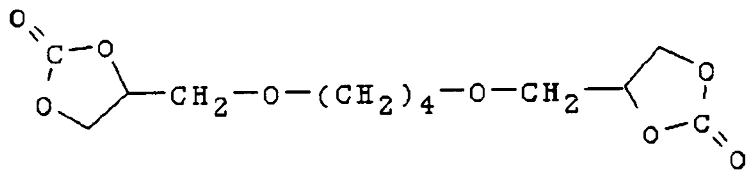

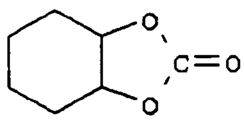

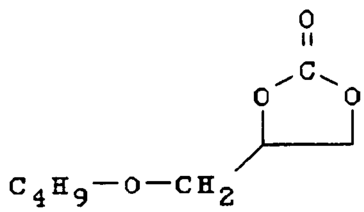

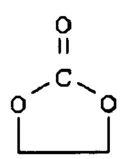

According to the process of the invention, the precursor carbonate is first directly fluorinated to provide a fluorinated carbonate intermediate. This direct fluorination step comprises contacting a cyclic or acyclic hydrocarbon carbonate (or a partially-fluorinated or partially-chlorinated analog thereof) with fluorine gas in a reactor maintained at a controlled temperature, which can be generally in the range of from about -20°C to +100°C, preferably from about 0°C to 50°C. The amount of fluorine gas utilized can be generally from about 1.0 to about 2.0 moles per mole of hydrogen atom replaceable by fluorine (depending upon whether a perfluorinated or a partially-fluorinated carbonate product is desired), preferably from about 1.0 to about 1.3 moles of fluorine per mole of replaceable hydrogen atoms. Where partially-fluorinated products are desired, lesser amounts of fluorine can be used. The fluorine gas is preferably diluted with an inert gas such as nitrogen or argon. If desired, the direct fluorination step can be carried out in the presence of a hydrogen fluoride scavenger, e.g., sodium fluoride.

Although a solid-phase process can be used, the direct fluorination step of the process of the invention is preferably a liquid-phase process (as described in said International Publication Nos. WO 90/03353 and WO 90/06296), which can be carried out in one of several ways. First, the direct fluorination step can be carried

out as a liquid-phase process in a "batch" manner, whereby the reactor is charged with a batch of the fluorinatable precursor carbonate (either neat or dissolved or dispersed in an inert, halogenated liquid or a fluorine-reactive liquid) and the inert liquid reaction medium to provide a very dilute concentration of the starting material, e.g., up to about 10% by weight, and then the fluorine gas (preferably diluted with inert carrier gas) is continuously bubbled through the solution or dispersion of the starting material, by-product hydrogen fluoride being continuously removed from the reactor as a gas (along with the unreacted fluorine gas), which removal is preferably aided by the flow of inert carrier gas through the reactor.

Alternatively, the direct fluorination step can be carried out as a liquid-phase process in a "semi-continuous" manner, with the carbonate starting material (either neat or dissolved or dispersed in a liquid, such as an inert halogenated liquid or a fluorine-reactive liquid) continuously pumped or otherwise fed as a gas, liquid, or solid into the reactor containing inert liquid reaction medium, e.g., for a two-liter reactor, at a rate of about 2 to 20 g/hr into 1500 mL of inert liquid, as the fluorine gas (preferably diluted with inert gas) is bubbled through the reaction mixture, e.g., at a fluorine flow rate of about 50 to 500 mL/min and an inert gas flow rate of about 200 to 2000 mL/min., by-product hydrogen fluoride and unreacted fluorine being continuously removed from the reactor, which removal is preferably aided by the inert carrier gas.

The direct fluorination step can also be carried out as a liquid-phase process in a "continuous" manner, with the starting material (either neat or dissolved or dispersed in an inert halogenated liquid or a fluorine-reactive liquid) continuously fed into the reactor containing inert liquid reaction medium, as described above, as the fluorine gas (preferably diluted with inert gas) is bubbled through the liquid reaction mixture. The solution or dispersion of unreacted and reacted starting material and the stream of unreacted fluorine, hydrogen fluoride gas, and inert carrier gas are continuously removed from the reactor. Separations can be made, if desired, to recover the fluorinated carbonate intermediate product, and, if also desired, the unreacted fluorine and unreacted starting material can be recycled. The amount of inert liquid medium in the reactor can be maintained at a constant level by addition of recycled or fresh liquid.

In general, the continuous addition of carbonate starting material is preferred and generally provides a higher yield, better product quality, and more efficient use of fluorine, though the batch mode has similar advantages if the "polishing" finishing step (described below) is used.

Suitable liquids for use as inert reaction media in a liquid-phase direct fluorination are those which can function as solvents or dispersants for the starting material and which do not react appreciably with diluted fluorine, that is, media which are relatively inert to fluorine, at the temperatures utilized. The concentration of the starting material in the inert reaction medium is relatively low so as to more easily control the reaction temperature. Examples of liquids useful as such reaction media include perfluoroalkanes such as perfluorinated pentanes, hexanes, heptanes, octanes, and decalins, perfluoroethers such as Fluorinert™ FC-75, Krytox™, and Fomblin™, perfluorotrialkylamines such as Fluorinert™ FC-40, chloro-fluorocarbons such as Freon™ 113, 1,1,2-trichloro-trifluoroethane, and Freon™ 11, fluorotrichloromethane, chlorofluoroethers such as 2,5,5-trichloroperfluoro-2-butyl tetrahydrofuran, perfluorobis(chloroethyl)ether, and perfluoropolyepichlorohydrin, perfluoroalkanesulfonyl fluorides such as perfluoro-1,4-butanedisulfonyl fluoride and perfluorobutanesulfonyl fluoride, and mixtures thereof. These inert media are conveniently used at atmospheric pressure. Lower molecular weight members of the above classes can also be used, but elevated pressures are then required to provide a liquid phase. In some cases it may be feasible to use the fluorinated carbonate intermediate product as a reaction medium, use of which may render unnecessary any desired separation of fluorinated product from reaction medium. Liquids suitable for use in diluting the starting material (prior to its addition to the inert liquid reaction medium) include the inert liquids described above as well as liquids which may to some degree react with dilute fluorine such as, for example, carbon tetrachloride, chloroform, and fluorinated alkanes containing one or two hydrogens, or materials which contain little or no halogen but in themselves are fluorinatable to useful products.

The reactor used in the direct fluorination step of the process of this invention can be equipped with a cooling jacket or internal cooling coils to control the temperature, a stirrer to vigorously agitate the reaction mixture as the fluorine gas is bubbled through it, and, if volatilized reaction medium and/or low boiling fluorinated product are to be recovered, a reflux condenser. When the direct fluorination is carried out by means of the above-described, preferred liquid-phase process, the reactor temperature will generally be maintained at a temperature in the range of about -20°C to about 100°C, preferably about 0°C to about 50°C, sufficient to volatilize the hydrogen fluoride by-product and with the aid of the flowing inert gas cause the purging of the by-product hydrogen fluoride from the fluorination reactor as it is generated. The design and temperature of the condenser should be such as to minimize or prevent the hydrogen fluoride from returning to the reactor, e.g., either by selective condensation of the inert liquid reaction medium and other organic substances, allowing the hydrogen fluoride to pass through the condenser, or by total condensation into a separate vessel of hydrogen fluoride, inert liquid reaction medium, and other organic substances followed by separation of the

hydrogen fluoride as the upper liquid phase and, if desired, recycle of the lower liquid phase. In some cases, a third (middle) phase may be formed containing, for example, precursor carbonates and partially-fluorinated carbonates. This phase may also be recycled if desired. The inert carrier gas flow rate sufficient for effective removal of hydrogen fluoride varies according to the reactor and condenser geometry. However, we have observed that a rate of about 1300 mL/min of 20% fluorine in nitrogen in a reactor containing 1.5 liters of Freon™ 113 at 20°C connected to a condenser consisting of about 6 meters of coiled 1.27 cm diameter stainless steel tubing at -25°C gives high yields of fluorinated product. Fluorine is preferably used at a concentration of about 5 to 50 volume %, more preferably about 10 to 25 volume %, in an inert gas such as, for example, nitrogen, argon, helium, $CF_4$, or $SF_6$, and is maintained in stoichiometric excess throughout the fluorination, for example, at an excess of up to 40% or higher. Pure fluorine can also be used but is not preferred, due to considerations of both safety and economy.

When perfluorinated carbonate intermediate product is sought by the practice of this invention, the semi-continuous and continuous liquid-phase fluorination methods yield perfluorinated product which may contain small amounts of fluorinated material having one or a few residual hydrogen atoms, but the product is essentially fully fluorinated, i.e., perfluorinated, with a residual hydrogen content of less than about 0.4 mg/g and generally less than about 0.1 mg/g. The batch process, however, gives product with a somewhat higher residual hydrogen content, e.g., about 7 mg/g. Partially-fluorinated products obtained by these methods are those with a residual hydrogen content generally greater than about 0.4 mg/g, e.g., up to about 10 mg/g.

The liquid reaction product obtained in a liquid-phase direct fluorination step can be distilled to remove the inert liquid reaction medium and any low-boiling by-products , and any residual hydrogen content and traces of undesired derivatives can be essentially completely removed upon treating the distillate at elevated temperature, for example, at 50°C or higher, with fluorine, preferably diluted with an inert gas such as nitrogen, to, in a sense, "polish" the product, the resulting hydrogen fluoride and any undesired volatile fluorinated derivatives being removed along with the unreacted fluorine gas used in this polishing technique.

After the direct fluorination step is carried out, the resulting perfluorinated or partially-fluorinated carbonate intermediate product is combined with a reactive nucleophile in an amount sufficient to effect cleavage of the carbonate, generally an amount ranging from a catalytic amount to a stoichiometric excess, depending upon the chosen nucleophile and the nature of the carbonate. The carbonate is allowed to react with the nucleophile to form a perfluorinated or partially-fluorinated functional compound. In some cases a solvent may be necessary to solubilize the reactants and to aid in heat transfer. For example, polar, aprotic solvents, such as dimethylformamide, glymes, acetone, and the like, can be used for such purpose when fluoride ion is the nucleophile. The reaction of the carbonate and the nucleophile can be facilitated by the application of heat and/or base.

If desired, prior to combination with the nucleophile, the carbonate can be removed from the fluorination reactor, e.g., by draining, and isolated, e.g., by fractional distillation. Preferably, however, the crude product of the direct fluorination step is kept in the reactor and the nucleophile added thereto and allowed to react with the carbonate. In some cases, an intermediate reaction product of the carbonate and nucleophile having the structure R-O-C(O)-Z, where Z represents the nucleophilic moiety, can be isolated if desired. Examples of such an intermediate product include fluoroformates and partially-fluorinated carbamates and carbonates. Otherwise, the reaction can be carried out to produce the desired final product, a perfluorinated or partially-fluorinated functional compound, which can then be isolated, if desired, by a method such as fractional distillation.

Nucleophiles which can be utilized in the process of the invention are those which are capable of reacting with the carbonate to effect cleavage thereof. Examples of such nucleophiles include water; alcohols such as methanol and ethanol; alkali metal fluoride salts such as sodium fluoride, cesium fluoride, and potassium fluoride; amines such as primary and secondary amines; ammonia; thiols; and other metal salts such as potassium thiocyanide and sodium cyanide. Representative schemes for the reaction of perfluorinated carbonates with various nucleophiles to provide perfluorinated functional compounds according to the process of the invention are shown below as Reactions A-1 to A-4, B-1 to B-4, and C-1 to C-4:

$CF_3\!-\!CF_2\!-\!CF_2\!-\!O\!-\!C(O)\!-\!O\!-\!CF_2\!-\!CF_2\!-\!CF_3$

$$\xrightarrow{3H_2O} 2CF_3\!-\!CF_2\!-\!C(O)\!-\!OH \;+\; CO_2 \;+\; 4HF \qquad \text{A-1}$$

$$\xrightarrow{4ROH} 2CF_3\!-\!CF_2\!-\!C(O)\!-\!OR \;+\; RO\!-\!C(O)\!-\!OR \;+\; 4HF \qquad \text{A-2}$$

$$\xrightarrow{4RNH_2} 2CF_3\!-\!CF_2\!-\!C(O)\!-\!NHR \;+\; RNH\!-\!C(O)\!-\!NHR \;+\; 4HF \qquad \text{A-3}$$

$$\xrightarrow{\text{catalytic amount of } F^-} 2CF_3\!-\!CF_2\!-\!C(O)\!-\!F \;+\; F\!-\!C(O)\!-\!F \qquad \text{A-4}$$

$(CF_3)_2CF\!-\!O\!-\!C(O)\!-\!O\!-\!CF(CF_3)_2$

$$\xrightarrow{1H_2O} 2(CF_3)_2C{=}O \;+\; CO_2 \;+\; 2HF \qquad \text{B-1}$$

$$\xrightarrow{2ROH} 2(CF_3)_2C{=}O \;+\; RO\!-\!C(O)\!-\!OR \;+\; 2HF \qquad \text{B-2}$$

$$\xrightarrow{2RNH_2} 2(CF_3)_2C{=}O \;+\; RNH\!-\!C(O)\!-\!NHR \;+\; 2HF \qquad \text{B-3}$$

$$\xrightarrow{\text{catalytic amount of } F^-} 2(CF_3)_2C{=}O \;+\; F\!-\!C(O)\!-\!F \qquad \text{B-4}$$

$$(CF_3)_3C{-}O{-}C(O){-}O{-}C(CF_3)_3$$

$$\xrightarrow{1H_2O} 2(CF_3)_3C{-}OH \quad + \quad CO_2 \qquad \text{C-1}$$

$$\xrightarrow{2ROH} 2(CF_3)_3C{-}OH \quad + \quad RO{-}C(O){-}OR \qquad \text{C-2}$$

$$\xrightarrow{2RNH_2} 2(CF_3)_3C{-}OH \quad + \quad RNH{-}C(O){-}NHR \qquad \text{C-3}$$

$$\xrightarrow{2F^-} 2(CF_3)_3C{-}O^- \quad + \quad F{-}C(O){-}F \qquad \text{C-4}$$

Functional compounds which can be prepared by the process of the invention include perfluorinated and partially-fluorinated carboxylic acid fluorides, esters, carboxylic acids, ketones, amides, and alcohols. Perfluorinated and partially-fluorinated carboxylic acids, as well as their derivatives such as salts, amides, esters, and acid fluorides, are useful as surface active agents and as pharmaceutical intermediates. Perfluorinated and partially-fluorinated ketones (such as hexafluoroacetone) can be used to prepare polymer intermediates (such as bisphenol AF) and are also useful as heat transfer agents. Perfluorinated and partially-fluorinated alcohols can be used as heat transfer agents, refrigerants, solvents, dielectric fluids, insulating fluids, and chemical intermediates. In addition, perfluorinated and partially-fluorinated difunctional compounds, such as those having both carboxylic acid functionality and alcohol functionality, are useful as reactants in condensation polymerization processes. Such multifunctional compounds can also be used as emulsifiers and as intermediates in the preparation of polymeric coatings.

This invention is further illustrated by the following examples, but the particular materials and amounts thereof recited in these examples, as well as other conditions and details, should not be construed to unduly limit this invention.

## Example 1

This example describes the preparation of hexafluoroacetone by the semi-continuous, liquid-phase direct fluorination of diisopropyl carbonate and the subsequent reaction of the perfluorinated product with cesium fluoride.

Diisopropyl carbonate was prepared by the reaction of phosgene with excess isopropanol in the presence of base. The direct fluorination of diisopropyl carbonate was carried out in a two-liter, jacketed vessel of Monel™ metal equipped with a magnetic drive agitator, a gas feed line, an organic reactant feed line, and a reflux condenser. The gas feed line was a 0.3 cm diameter tube reaching to a point below the bottom impeller of the agitator. The organic reactant feed line was a 0.15 cm diameter tube connected to a syringe pump. The reflux condenser consisted of about 6 m of two coiled concentric tubes, the inner tube having a 1.27 cm diameter and the outer tubing having a 2.54 cm diameter. Gases from the reactor were cooled in the inner tube by refrigerant, ethylene glycol-water, flowing in the annulus between the two tubes. The reactor was charged with 3095 g of Freon™113 (available from DuPont), which was stirred while the reactor was purged with 1000 mL/min of nitrogen for about five minutes. The gas stream was changed to a mixture of 250 mL/min of fluorine and 1000 mL/min of nitrogen. After seven minutes, the feeding of a solution of 92 g of diisopropyl carbonate diluted to 289 mL with Freon™113 was initiated and maintained at a constant rate for about 19.6 hours. The temperature of the reaction mixture was maintained at 17-18°C throughout the feeding. After nitrogen purging for about one additional hour, the crude product was drained from the reactor.

Fractional distillation of the crude drainings using a column with approximately three theoretical plates gave a cut boiling at 82-87°C. $^{19}F$ and $^{1}H$ NMR analyses on this cut indicated that it consisted predominately of perfluoro(diisopropyl carbonate),

$$(CF_3)_2CF{-}O{-}C(O){-}O{-}CF(CF_3)_2.$$

A dry flask was charged with a 9.5 g sample of the above-described cut boiling at 82-87°C and 20 mL of dry diglyme. 1.31 g of anhydrous cesium fluoride was added. The gases evolved were swept with dry nitrogen through water to yield hexafluoroacetone hydrate and some perfluoro(diisopropyl carbonate).

Example 2

This example describes the preparation of perfluoro(butoxypyruvic acid) hydrate by the semi-continuous, liquid-phase direct fluorination of butoxymethylethylene carbonate and the subsequent hydrolysis of the perfluorinated product.

Butoxymethylethylene carbonate was prepared by the catalyzed addition of $CO_2$ to butyl glycidyl ether essentially by the procedure of M. V. Mikheev et al. (J. Org. Chem., USSR 1983, 19, 436-38), using acetone as the solvent. Direct fluorination of this cyclic carbonate was carried out in essentially the manner described in Example 1. A solution of 20 g of butoxymethylethylene carbonate diluted to 200 mL in Freon™113 was fed to the two-liter fluorination reactor over 3.8 hours. Distillation of the crude product mixture gave predominately perfluoro(butoxymethylethylene carbonate) boiling at 112-125°C.

Perfluoro(butoxymethylethylene carbonate) was hydrolyzed, as shown in the following reaction, bY heating it with water in a sealed stainless steel cylinder at 70-75°C overnight.

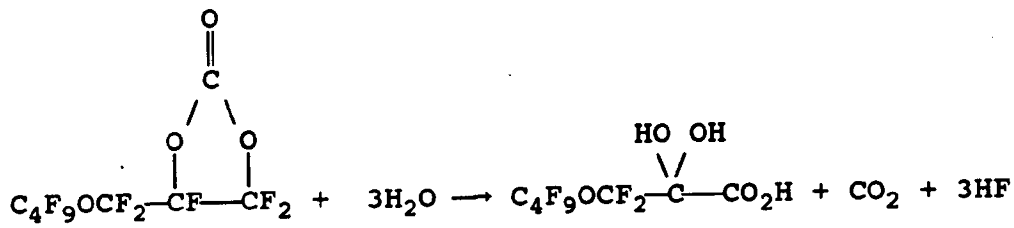

The hot cylinder was vented and gentle suction applied to remove residual water. The residue in the cylinder was predominately crude perfluoro(butoxypyruvic acid) hydrate, having a melting point of 113-118°C. Vacuum sublimation at 100°C/0.1 Torr gave colorless crystals melting at 118-119°C. $^{19}F$ and $^{1}H$ NMR and infrared spectroscopic analyses confirmed the identity of the hydrated ketoacid to be that shown in the above-illustrated reaction.

Example 3

This example describes the preparation of ethyl perfluoro(ethoxy acetate) and ethyl perfluoro(2-ethoxyethyl carbonate) by the semi-continuous, liquid-phase direct fluorination of bis(2-ethoxyethyl) carbonate and the subsequent ethanolysis of the perfluorinated product.

A 100 g sample of bis(2-ethoxyethyl) carbonate was fluorinated in a 2-L fluorination reactor in essentially the manner described in Example 1. After the fluorination was completed but before the reactor was drained, 100 g of absolute ethanol was fed over 1 hour with agitation and nitrogen purge to remove HF formed in the ethanolysis. The reactor was then drained, and the crude product was washed with aqueous base, dried over $CaCl_2$, filtered, and distilled. Ethyl perfluoro(ethoxy acetate) was collected boiling at 85-110°C. A higher boiling fraction contained a mixture of ethyl perfluoro(ethoxy acetate) and ethyl perfluoro(2-ethoxyethyl carbonate). The distillation residue was predominately ethyl perfluoro(2-ethoxyethyl carbonate), as confirmed by $^{19}F$ and $^{1}H$ NMR.

Example 4

This example describes the preparation of perfluoro(methoxyacetic acid) by the semi-continuous, liquid-phase direct fluorination of bis(2-methoxyethyl) carbonate and the subsequent hydrolysis of the perfluorinated product.

$$(CH_3OC_2H_4O)_2CO \rightarrow (CF_3OC_2F_4O)_2CO \rightarrow CF_3OCF_2CO_2H$$

155 g of bis(2-methoxyethyl) carbonate was fluorinated in essentially the manner described in Example 1. Fractional distillation of the crude product left a residue of a lightly-colored, somewhat cloudy liquid. This residue was vacuum transferred at 2.5 Torr, and the condensate, a clear, colorless liquid, was shown by gas chromatography to be perfluoro[bis(2-methoxyethyl)carbonate].

8.63 g of this perfluoro carbonate and 1.12 g of water were heated in a sealed stainless steel cylinder at 100°C for 15.5 hours to hydrolyze the carbonate. The cylinder was vented to atmospheric pressure while it was

still hot. After cooling the cylinder, the liquid recovered from the cylinder was analyzed by gas chromatography and $^{19}F$ NMR, which indicated that the major component of this liquid was perfluoro(methoxyacetic acid).

Example 5

This example describes the preparation of perfluoro(2-methoxyethoxyacetic acid) by the semi-continuous, liquid-phase direct fluorination of bis[2-methoxy(2-ethoxyethyl)] carbonate and the subsequent hydrolysis of the perfluorinated product.

$$[CH_3O(C_2H_4O)_2]_2CO \rightarrow [CF_3O(C_2F_4O)_2]_2CO \rightarrow CF_3OC_2F_4OCF_2CO_2H$$

Bis[2-methoxy(2-ethoxyethyl)] carbonate was fluorinated in essentially the manner described in Example 1. Fractional distillation of the crude reaction mixture left a lightly-colored, clear liquid and a trace of gummy, insoluble material as the crude product. Analysis of the clear liquid product by gas chromatography showed it to be predominately perfluoro(bis[2-methoxy(2-ethoxyethyl)] carbonate). A sample of the clear liquid product was purified by vacuum distillation at 55-65°C/6.5 Torr.

A 1.84 g sample of the clear liquid product was hydrolyzed, essentially as in Example 4, to give a liquid product which was distilled bulb-to-bulb at 100-105°C/100 Torr, yielding 1.21 g of colorless distillate. The major component of this distillate was shown to be perfluoro-(2-methoxyethoxyacetic acid) by $^{19}F$ NMR.

Example 6

This example describes the preparation of perfluoropinacol by the solid-phase direct fluorination of pinacol carbonate and the subsequent hydrolysis of the perfluorinated product.

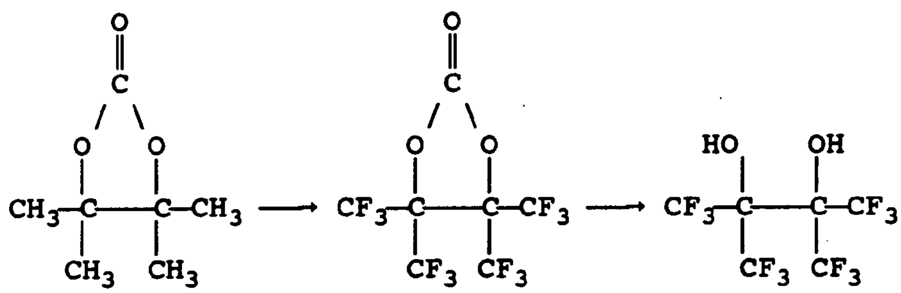

Pinacol carbonate, m.p. 176-9°C, was prepared by addition of a solution of bis(trichloromethyl) carbonate (triphosgene) in dichloromethane to an anhydrous mixture of equimolar amounts of pinacol and pyridine dissolved in toluene and dichloromethane.

Fluorination of pinacol carbonate was accomplished by the "solids fluorination" method described in U.S. Pat. Nos. 4,755,567 and 4,859,747 (Bierschenk et al.). Thus, an intimate mixture of 14.5 g of pinacol carbonate and 75 g of sodium fluoride powder was tumbled in a solids fluorination reactor at room temperature, as fluorine gas diluted with nitrogen was introduced. Gases leaving the reactor passed through a cold trap (-78°C), then through a column of alumina, and finally through a sensor to determine oxygen concentration (from reaction of alumina with fluorine).

The initial fluorine concentration in the gas introduced to the reactor was 10%. Over a period of about 24 hours, this concentration was increased to 40%. The oxygen content of the gas stream measured at the oxygen sensor stayed very low (<2%) through most of this time, then rose to 17% near the end of this run. Fluorine flow was shut down, and the reactor was purged with nitrogen. Gas chromatography and $^{19}F$ NMR analysis of the contents of the cold trap indicated that perfluoro(pinacol carbonate), $C_7F_{12}O_3$, represented about half of the contents. Lesser amounts of related partially-fluorinated carbonates were also present. No attempt was made to separate these closely related components.

This mixture of carbonate esters was heated with a stoichiometric excess of water, essentially as described in Example 4. The hydrolysis of the perfluoro(pinacol carbonate) component to perfluoropinacol was about 50% complete after 42 hours at 100°C, as shown by $^{19}F$ NMR and gas chromatography.

Example 7

This example describes the preparation of perfluoro(2,2-dihydroxydecanoic acid) by the semi-continuous, liquid-phase direct fluorination of n-octyl-ethylene carbonate and the subsequent hydrolysis of the perfluor-

inated product.

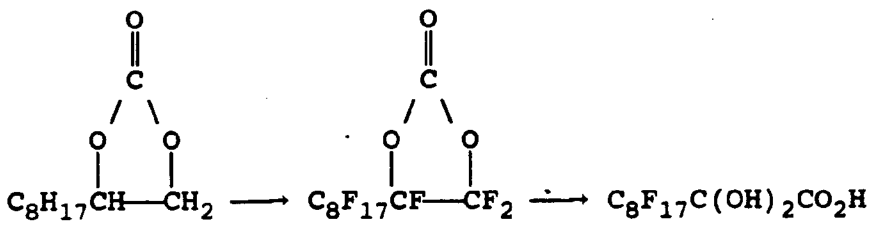

n-Octylethylene carbonate was prepared by the carbonation of 1,2-epoxydecane, and 100 g of the carbonate was solution fluorinated in essentially the manner described in Example 1. Distillation gave perfluoro(n-octylethylene carbonate) (as confirmed by $^{19}F$ NMR) having a boiling point range of 182-186°C. This material crystallized (m.p. 30-32°C) on cooling and slowly hydrolyzed on exposure to air at room temperature.

Controlled hydrolysis with a stoichiometric amount of water produced perfluoro(2,2-dihydroxydecanoic acid), a colorless crystalline solid with a broad melting point range (118-160°C). Purification was attempted by vacuum sublimation (100°C/0.01 Torr), but this did not change this characteristic melting behavior.

Example 8

This example describes the preparation of perfluoro(1,1-dihydroxycyclohexane) by the semi-continuous, liquid-phase direct fluorination of methyl cyclohexyl carbonate and the subsequent hydrolysis of the perfluorinated product.

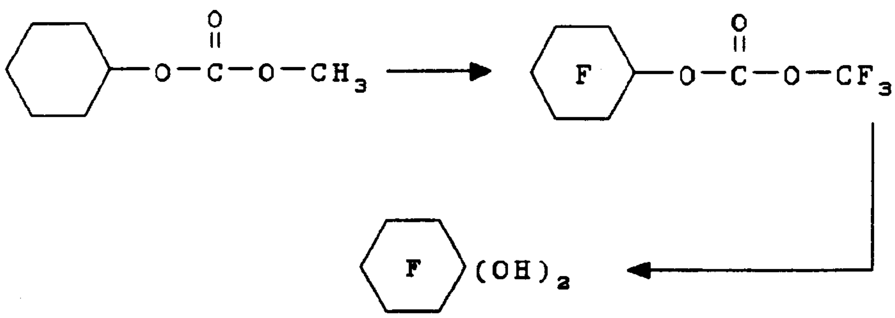

(The "F" within the depicted cyclohexane rings indicates that the rings are perfluorinated.)

Methyl cyclohexyl carbonate was prepared by the addition of methyl chloroformate to equimolar amounts of cyclohexanol and pyridine in dichloromethane. Fluorination of 43 g of this carbonate was carried out in essentially the manner described in Example 1. Distillation gave predominately perfluoro(methyl cyclohexyl carbonate) (boiling point range of 110-115°C), as indicated by gas chromatographic and $^{1}H$ and $^{19}F$ NMR analysis.

Hydrolysis of this product mixture, essentially as in Example 4 above, produced predominately perfluoro(1,1-dihydroxycyclohexane), which was purified by bulb-to-bulb distillation at 100-110°C/335 Torr.

Example 9

This example describes the preparation of perfluoro(1,1-dihydroxycyclohexane) by the semi-continuous, liquid-phase direct fluorination of diphenyl carbonate and the subsequent hydrolysis of the perfluorinated product.

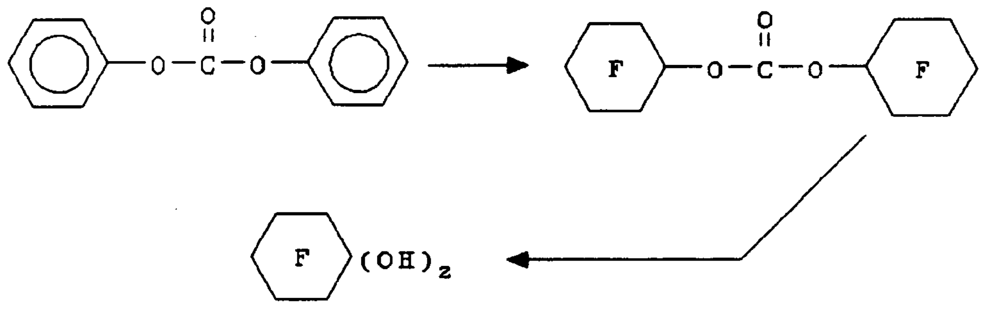

A solution of 50 g of diphenyl carbonate in 110 g of chloroform was fluorinated in essentially the manner described in Example 1. Distillation gave predominately perfluoro(dicyclohexyl carbonate), boiling at 168-180°C, and minor amounts of chlorine-containing carbonates, as confirmed by gas chromatography and $^{19}F$ NMR.

Hydrolysis, essentially as in Example 4, of 8.0 g of this mixture produced a low melting solid that was distilled bulb-to-bulb at reduced pressure (boiling point range of 115-120°C/335 Torr). The distillate, a clear, colorless slush, was a mixture of predominately perfluoro(1,1-dihydroxycyclohexane), with minor amounts of chlorinated derivatives thereof.

Various modifications and alterations of this invention will become apparent to those skilled in the art without departing from the scope and spirit of this invention.

## Claims

1. A process for the preparation of fluorinated functional compounds comprising the following steps:
   (a) directly fluorinating a fluorinatable cyclic or acyclic carbonate by contacting said carbonate with fluorine gas in a temperature-controlled reactor to fluorinate said carbonate; and
   (b) combining the resulting fluorinated carbonate with a reactive nucleophile and allowing said fluorinated carbonate and said nucleophile to react to form a fluorinated functional derivative of said fluorinated carbonate.

2. The process of Claim 1 wherein said fluorinated carbonate is perfluorinated.

3. The process of Claim 1 wherein said fluorinated carbonate is partially-fluorinated.

4. The process of Claim 1 wherein said fluorinatable cyclic or acyclic carbonate is represented by the formula R-O-C(O)-O-R', wherein R and R' are independently selected from the group consisting of primary, secondary, and tertiary aliphatic groups; alkyl-substituted and unsubstituted cycloaliphatic groups; aryl groups; alkylene groups which are such that R and R' are bonded together to form a cyclic structure; and combinations of such groups; and wherein R and/or R' can contain one or a plurality of carbon-bonded, catenary heteroatoms, can contain additional carbonate functionality, and can be partially-fluorinated or partially-chlorinated; and wherein R or R' can be fully-fluorinated.

5. The process of Claim 4 wherein R and R' are independently selected from the group consisting of primary, secondary, and tertiary aliphatic groups having from 1 to 20 carbon atoms; alkyl-substituted and unsubstituted cycloaliphatic groups having from 3 to 20 carbon atoms; aryl groups having from 6 to 14 carbon atoms; alkylene groups having from 1 to 20 carbon atoms and being such that R and R' are bonded together to form a cyclic structure; and combinations of such groups; and wherein R and/or R' can contain one or a plurality of carbon-bonded, catenary heteroatoms.

6. The process of Claim 1 wherein said fluorinatable cyclic or acyclic carbonate is selected from the group consisting of diisopropyl carbonate, bis(2-ethoxyethyl) carbonate, bis(2-methoxyethyl) carbonate, bis[2-methoxy(2-ethoxyethyl)] carbonate, n-octylethylene carbonate, butoxymethylethylene carbonate, pinacol carbonate, methyl cyclohexyl carbonate, and diphenyl carbonate.

7. The process of Claim 1 wherein said fluorinatable cyclic or acyclic carbonate is directly fluorinated in a liquid-phase process by directly contacting a diluted solution or dispersion of said fluorinatable carbonate

in a normally liquid, inert medium with fluorine gas, optionally diluted with an inert gas, in a temperature-controlled reactor to fluorinate said carbonate either in the presence of a hydrogen fluoride scavenger or alternatively at a temperature and a flow rate of inert gas (if used) sufficient to volatilize the resulting by-product hydrogen fluoride, removing said hydrogen fluoride from said reactor as it is produced.

**8.** The process of Claim 1 wherein said reactive nucleophile is selected from the group consisting of water, alcohols, thiols; amines, ammonia, and alkali metal fluoride salts.

**9.** The process of Claim 1 wherein said fluorinated functional compound is selected from the group consisting of perfluorinated and partially-fluorinated carboxylic acids, esters, ketones, alcohols, amides, and carboxylic acid fluorides.

**10.** The process of Claim 1 wherein said step (a) is carried out by directly fluorinating diisopropyl carbonate by directly contacting a diluted solution or dispersion of said carbonate in a normally liquid, inert medium with fluorine gas, optionally diluted with an inert gas, in a temperature-controlled reactor to perfluorinate said carbonate either in the presence of a hydrogen fluoride scavenger or alternatively at a temperature and a flow rate of inert gas (if used) sufficient to volatilize the resulting by-product hydrogen fluoride, removing said hydrogen fluoride from said reactor as it is produced; and wherein said step (b) is carried out by combining the resulting perfluorinated carbonate with an alkali metal fluoride salt and allowing said perfluorinated carbonate and said salt to react to form hexafluoroacetone.

European Patent Office

# EUROPEAN SEARCH REPORT

Application Number

EP 93 40 0360

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | FR-A-2 647 107 (RHONE-POULENC CHIMIE) * examples 9,11 * | 1 | C07C49/167 C07C59/115 C07C69/708 |
| A | FR-A-2 647 106 (RHONE POULENC CHIMIE) * example 4 * | 1 | C07C59/135 C07C31/44 C07C59/21 |
| A,D | US-A-3 455 954 (J.H. PRAGER) | | C07C41/42 C07C45/54 C07C51/00 C07C29/09 C07C67/08 //C07C59/215 C07C49/643 C07B39/00 C07B41/00 C07C69/96 C07C68/06 C07D317/42 C07D317/36 |
| | | | TECHNICAL FIELDS SEARCHED (Int. Cl.5 ) |
| | | | C07C C07D |

The present search report has been drawn up for all claims

| Place of search | Date of completion of the search | Examiner |
|---|---|---|
| BERLIN | 10 JUNE 1993 | VAN AMSTERDAM L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)